# EUROPÄISCHE PATENTSCHRIFT

(11) **EP 3 323 455 B1**
(45) Veröffentlichungstag und Bekanntmachung des Hinweises auf die Patenterteilung: **11.08.2021**
(21) Anmeldenummer: 16199873.7
(22) Anmeldetag: 21.11.2016
(51) Int. Cl.: A61M 11/00, A61M 15/00, B05B 12/18, B65D 83/20, B65D 83/30, A61M 16/06

(54) **INHALATIONSEINRICHTUNG ZUM ZWECKE DES INHALIERENS EINES TRÖPFCHENNEBELS**
INHALATION APPARATUS FOR THE PURPOSE OF INHALING A DROPLET MIST
DISPOSITIF D'INHALATION DE BROUILLARD DE GOUTTELETTES

(43) Veröffentlichungstag der Anmeldung: 23.05.2018
(73) Patentinhaber: Aptar Radolfzell GmbH, 78315 Radolfzell (DE)
(72) Erfinder: Greiner-Perth, Jürgen, 78244 Gottmadingen (DE)
(74) Vertreter: Patentanwaltskanzlei Cartagena

(56) Entgegenhaltungen:
- EP-A1- 1 743 671
- EP-A1- 3 095 522
- EP-A1- 3 275 492
- EP-A1- 3 275 555
- EP-A1- 3 275 558
- WO-A1-2005/084735
- WO-A1-2009/023210
- WO-A1-2010/103227
- WO-A1-2011/147714
- WO-A1-2014/140774
- WO-A2-02/074372
- WO-A2-2015/149922
- WO-A2-2015/155606
- CN-U- 203 400 384
- US-A- 4 452 239
- US-A- 5 437 267

## Beschreibung

### ANWENDUNGSGEBIET UND STAND DER TECHNIK

Die Erfindung betrifft eine Inhalationseinrichtung zum Zwecke des Inhalierens eines Tröpfchennebels durch einen Benutzer.

Derartige Inhalationseinrichtungen dienen dem Zweck, den Atemwegen und der Lunge des Benutzers eine Flüssigkeit in vernebelter Form zuzuführen. Die dabei verwendete Flüssigkeit ist eine pharmazeutische Flüssigkeit, die der Linderung von Atemwegsbeschwerden und Atembeschwerden dient. Die Flüssigkeit kann aktive pharmazeutische Wirkstoffe enthalten oder darauf ausgerichtet sein - wie beispielsweise Salzwasser - ohne solche aktiven Substanzen zur Linderung der Beschwerden beizutragen.

Die Erfindung betrifft insbesondere mobile Inhalationseinrichtungen, bei denen die Flüssigkeit manuell oder durch einen Druckspeicher druckbeaufschlagt und einer Düseneinrichtung zugeführt wird, die den Tröpfchennebel hieraus erzeugt.

Aus der nachveröffentlichten EP 3095522 A1 ist eine gattungsgemäße Inhalationseinrichtung bekannt. Ein wesentliches Problem im Bereich der gattungsgemäßen Inhalationseinrichtung liegt in Erzeugung und Zuführung ausreichend feiner Tröpfchen im Tröpfchennebel. Bei Düsenanordnungen, die ohne mechanisch bewegliche Teile einen Tröpfchennebel erzeugen, hängt die Tröpfchengröße maßgeblich vom Druck der zugeführten Flüssigkeit ab. Gerade bei den eingangs bereits genannten mobilen Inhalationseinrichtungen, die mobil und ohne elektrische Aggregate verwendet werden und bei denen es schwierig ist, einen Druck der zugeführten Flüssigkeit oberhalb von 5 bar zu realisieren, sind nur schwer zur Erzeugung atemwegsgängiger Tröpfen geeignet. Hinzu kommt die Problematik, dass die ausgebrachten Tröpfchen des erzeugten Tröpfchennebel aufgrund des Luftwiderstandes zur Agglomeration zu größeren Tröpfchen neigen, noch bevor sie in die Atemwege gelangt sind.

Aus den ebenfalls nachveröffentlichten Dokumente EP 3 275 492 A1, EP 3 275 555 A1 und EP 3 275 558 A1 sind Inhalationseinrichtungen bekannt, bei denen ein Außenbauteil innenliegende flüssigkeitsführe Teile umgibt.

Die vorveröffentlichte WO 2011/147714 A1 ofenbart einen im Allgemeinen zylindrischer Aerosolinhalator, die einen Rauchartikel wie eine Zigarette simuliert. Dieser Inhalator umfasst ein zylindrisches Kanistergehäuse, ein zylindrisches Kanister mit der abzugebenden Flüssigkeit, einen Körper, ein Düsenelement und ein zylindrisches Mundstück, dass am Körper drehbar gelagert ist. Der Kanister enthält ein Ventil, das zum Austritt einer Dosis der Flüssigkeit zur Aerosolisierung im Düsenelement geöffnet wird. Der Körper nimmt das Düsenelement auf und umfasst einen Mittelabschnitt zum Antreiben des Düsenelements beim Drehen des Mundstücks. Wenn das Mundstück gedreht wird, so wird das Düsenelement angetrieben zur Öffnung des Ventils, womit eine Dosis der Flüssigkeit im Kanister ausgetragen wird, im Düsenelement aerosolisiert wird, und durch das Mundstück an einen Verbraucher verabreicht wird.

Die vorveröffentlichte WO 2015/149922 A2 offenbart einen Aerosolinhalator für ein Pferd, wo ein Aerosol mittels einer Düsenanordnung in einer relativ zu einer Mittelachse eines Reservoirs abgewinkelten Richtung erzeugt wird und durch eine weiter abgewinkelte Austragöffnung an ein Pferd verabreicht wird.

### AUFGABE UND LÖSUNG

Aufgabe der Erfindung ist es, eine Bauweise einer Inhalationseinrichtung zur Verfügung zu stellen, mit der sich eine Inhalationseinrichtung einfach anpassen lässt, insbesondere auch in Hinblick auf unterschiedlichen Ausgabearten und/oder die jeweils gewünschte Tröpfchengröße.

Erfindungsgemäß wird eine Gestaltung einer Inhalationseinrichtung vorgeschlagen, bei der die Inhalationseinrichtung eine Basis, umfassend einen Flüssigkeitsspeicher zur Lagerung von Flüssigkeit vor ihrem Austrag, und einen Austragkopf für die Abgabe der Flüssigkeit in Form eines Tröpfchennebels umfasst. Der Austragkopf weist eine Düsenanordnung zur Erzeugung des Tröpfchennebels auf. Die Inhalationseinrichtung umfasst ein Schaltventil, mittels dessen ein Zufuhrkanal vom Flüssigkeitsspeicher zur Düsenanordnung geöffnet und geschlossen werden kann.

Der Austragkopf ist um eine Hauptdrehachse gegenüber der Basis verdrehbar, wobei hierdurch das Schaltventil geöffnet und geschlossen werden kann.

Der hierbei vorgesehene Austragkopf umfasst ein Innengehäuse, welches mit der Basis verbunden ist und an welchem die Düsenanordnung zur Abgabe des Tröpfchennebels in einer zur Hauptdrehachse angewinkelten Abgaberichtung vorgesehen ist, und ein Außengehäuse, welches in einer definierten Drehstellungaufdas Innengehäuseaufsetzbarist. Das Innengehäuse und dasAußengehäuse sind formschlüssig in Hinblick auf eine Rotation um die Hauptdrehachse zusammenwirkend ausgebildet, so dass durch eine Drehbewegung des Außengehäuses gegenüber der Basis mittelbar auch eine Drehbewegung des Innengehäuses gegenüber der Basis erzielbar ist.

Das Außengehäuse verfügt über einen Austragkanal, der zur Nutzung als Atemstück oder zu Anbringung eines Atemstücks ausgebildet ist und der durch Aufsetzen des Außengehäuses derart relativ zur Düsenanordnung angeordnet wird, dass durch den Austragkanal hindurch der von der Düsenanordnung abgegebene Tröpfchennebel inhaliert werden kann.

Bei der vorgeschlagenen Gestaltung liegt die Besonderheit darin, dass der Austragkopf über ein Innengehäuse verfügt, welches auch die Düsenanordnung umfasst. Dieses ist gegenüber der Basis der Inhalationseinrichtung bestimmungsgemäß zum Öffnen und Schließen des genannten Schaltventils drehbar. Es ist also bestimmungsgemäß vorgesehen, dass das Innengehäuse durch Verdrehen gegenüber der Basis den Austragvorgang initiiert und durch entgegengesetztes Drehen wieder beendet.

Das Außengehäuse ist vorzugsweise in einer Richtungder Hauptachse auf das Innengehäuse aufgeschoben, wobei fluchtend mit der Abgaberichtung der Düsenanordnung ein Einlass auf der Innenseite des Außengehäuses vorgesehen ist, der in den Austragkanal führt, an dessen distalem Ende durch den Benutzer zum Zwecke des Inhalierens gesogen wird.

Die Hauptachse der Inhalationseinrichtung kann durch eine Hauptdrehachse, um die der Austragkopf gegenüber dem Flüssigkeitsspeicher drehbar ist, und/oder durch rotationssymmetrische Formgebung des flaschenartig ausgebildeten Flüssigkeitsspeichers gebildet sein. Wenn im Weiteren auf die Hauptachse Bezug genommen wird, handelt es sich wahlweise um die Hauptdrehachse oder die Symmetrieachse. Besonders vorteilhaft ist eine Gestaltung, bei denen diese Achsen identisch sind.

Die Modularität, die durch die genannte Gestaltung des Innengehäuses und des Außengehäuses erzielt wird, bietet mehrere Vorteile. So ist zum einen herstellerseitig möglich, in Abhängigkeit des auszubringenden Mediums unterschiedliche Außengehäuse einzusetzen. Da das Außengehäuse vorzugsweise jenes Bauteil ist, welches durch seine Formgebung die eingangs genannte Anwinkelung zwischen der Abgaberichtung und der Richtung des Austragkanals bestimmt, kann dieser Winkel durch Wahl eines von mehreren möglichen Außengehäusen variabel festgelegt werden. Die genannte Modularität gestattet es darüber hinaus, in Abhängigkeit des Verwendungszwecks und insbesondere der Beschwerden des Benutzers alternativ eine Atemmaske oder ein Mundstück zu verwenden, welche einstückig am Außengehäuse angeformt sind. Diese Flexibilität kann jedoch auch durch ein mehrstückiges Außengehäuse erzielt werden, welches im Weiteren noch erläutert wird.

Das Innengehäuse und das Außengehäuse weisen vorzugsweise miteinander zusammenwirkende Klemmbereiche innenseitig am Außengehäuse und außenseitig am Innengehäuse auf, im Bereich derer sie unter Bildung einer Presspassung kraftschlüssig haltend ineinander geschoben werden können. Die genannten Klemmbereiche führen vorzugsweise zu einem umlaufenden Klemmen zwischen Innengehäuse und Außengehäuse, so dass hierzum einen nicht in ungewollter Weise Luft eindringen kann und zum anderen ein ungewolltes Abfallen des Außengehäuses verhindert wird.

Das Außengehäuse weist erfindungsgemäß eine Mantelfläche auf. Außenseitig an dieser Mantelfläche ist ein Kanalstutzen vorgesehen, der den Austragkanal bildet und dessen Haupterstreckungsachse gegenüber der mittleren Abgaberichtung der Düsenanordnung angewinkelt ist. Vorzugsweise ist es eine Wandung des Kanalstutzens, die mit der mit der Abgaberichtung der Düsenanordnung fluchtenden Durchbrechung versehen ist. Der genannte Kanalstutzen ist also vorzugsweise asymmetrisch am Außengehäuse vorgesehen und kann insbesondere vorzugsweise eine in etwa tangentiale Ausrichtung haben.

Vorzugsweise ist ein Flüssigkeitsaufnahmeraum zur Aufnahme von Flüssigkeit vorgesehen, die sich nach Abgabe durch die Düsenanordnung innenseitig am Außengehäuse niederschlägt. Ein solcher Flüssigkeitsaufnahmeraum dient dem Zweck, dass sich niedergeschlagene Flüssigkeit automatisch hier sammelt und nicht ohne weiteres auslaufen kann. Hierzu ist der Flüssigkeitsaufnahmeraum vorzugsweise als eine Art vertiefter Aufnahmebereich vorgesehen, wenn die Inhalationseinrichtung in der üblichen Weise mit nach oben weisendem Austragkopf und nach unten weisendem Flüssigkeitsspeicher gehalten wird.

Insbesondere kann der Flüssigkeitsaufnahmeraum gemeinsam durch einen Innenwandung des Außengehäuses und eine Außenwandung des Innengehäuses gebildet werden. Es wird als vorteilhaft angesehen, wenn der Flüssigkeitsaufnahmeraum als Ringraum ausgebildet ist. Die Verwendung eines Flüssigkeitsaufnahmeraums, der durch das Außengehäuse und das Innengehäuse gemeinsam begrenzt wird, führt zu einem sehr einfachen Entleeren dieses Flüssigkeitsaufnahmeraums. Wenn nach der Verwendung der Inhalationseinrichtung das Außengehäuse vomInnengehäuse abgezogen wird, wird damit auch der Flüssigkeitsaufnahmeraum geöffnet, so dass die überschüssige Flüssigkeit gezielt weggeschüttet werden kann.

Der Kanalstutzen, der den Austragkanal bildet, kann in einem Mundstück oder in einer Atemmaske münden. Alternativ kann ein vom Kanalstutzen getrenntes Mundstück oder eine getrennte Atemmaske zur Ankoppelung an den Kanalstutzen vorgesehen sein. Wenn der Austragkanal als Mundstück ausgebildet ist, weist er vorzugsweise eine von einem kreisförmigen Querschnitt abweichende Querschnittsfläche auf, insbesondere eine in etwa ovale oder anderweitig längliche, die gut von den Lippen umschlossen werden kann. Durch die Möglichkeit, ein getrenntes Mundstück oder eine getrennte Atemmaske als Atemstück zu verwenden, welche insbesondere mittels einer Steckverbindung mit dem Kanalstutzen verbunden werden kann, ergibt sich die Möglichkeit, in Abhängigkeit des konkreten Anwendungszweckes und/oder der Beschwerden des Benutzers eine optimale Konfiguration herzustellen.

Der Flüssigkeitsspeicher ist vorzugsweise als Druckspeicher ausbildet, in dem die Flüssigkeit unter Überdruck vorgehalten wird. Er ist vorzugsweise zur Aufnahme von maximal 500 ml Flüssigkeit ausgelegt, insbesondere vorzugsweise von maximal 250 ml. Die Verwendung eines Druckspeichers ist von Vorteil, da hierdurch vergleichsweise hohe Drücke erzeugt werden können und diese insbesondere nutzerunabhängig vorliegen. Als besonders vorteilhaft wird ein System angesehen, bei dem im Druckspeicher ein Flüssigkeitsbeutel angeordnet ist, der durch komprimierte Luft außenseitig des Beutels druckbeaufschlagt wird. Es lassen sich auch alternative Gestaltungen mit einer durch den Benutzer erfolgenden Druckbeaufschlagung realisieren.

Das genannte Aufnahmevolumen des Flüssigkeitsspeichers ist jenes, welches bei mobilen Inhalatoren als besonders zweckmäßig angesehen wird.

Die Düsenanordnung kann eine Düsenplatte mit einer Mehrzahl von Düsenöffnungen zur Erzeugung des Tröpfchennebels aufweisen. Eine solche Düsenanordnung mit einer Düsenplatte stellt einen besonders einfachen Weg zur Erzeugung eines Tröpfchennebels dar. Vorzugsweise handelt es sich um eine Siliziumplatte. Weiterhin liegt die Anzahl der Düsenöffnungen vorzugsweise jenseits von 10 Düsenöffnungen, beispielsweise bei 20 oder mehr Düsenöffnungen, deren Durchmesser vorzugsweise im Bereich zwischen 2 µm und 200 µm liegt.

Wenngleich eine solche Düsenplatte als sehr vorteilhafter Weg zur Erzeugung des Tröpfchennebels angesehen wird, sind auch anderweitige Gestaltungen möglich. Hierzu gehört insbesondere auch die Verwendung einer Wirbelkammer, in die die Flüssigkeit derart einströmt, dass ein Drall entsteht, durch den die Flüssigkeit beim Austreten in Einzeltröpfchen zerrissen wird. Obwohl sich mit einer solchen Technik unmittelbar nur schwer eine Tröpfchenbildung realisieren lässt, bei der die Tröpfchen ausreichend atemwegsgängig sind, ist durch die beschriebene Umlenkung des Tröpfchennebels und die damit einhergehende Verfeinerung dennoch auch die Verwendung bei einer Inhalationseinrichtung möglich.

Der Austragkopf ist zum Zweck des Öffnens und Schließens des Schaltventils vorzugsweise in einer überlagerten rotativen und axialen Bewegunggegenüberder Basisverlagerbar. Hierfür ist insbesondere vorzugsweise zwischen der Basis und dem Austragkopf eine Kulissenführung mit einer helixabschnittsförmigen Kulisse vorgesehen. Diese Bauweise stellt einen besonders einfachen und störungsarmen Weg dar, eine Drehbewegung des Austragkopfes gegenüber der Basis zum Öffnen und Schließen eines Ventils zu nutzen. Das Schaltventil kann derart ausgebildet sein, dass es durch eine axiale Bewegung geöffnet und geschlossen wird. Durch die erzwungene Überlagerung einer rotativen und einer axialen Bewegung führt die genannte Drehbewegung dann zum Öffnen oder Schließen.

Der Flüssigkeitsspeicher der beschriebenen Inhalationseinrichtung ist vorzugsweise mit einer der folgenden Flüssigkeiten befüllt: einer salzhaltigen wässrigen Lösung, einer wässrigen Lösung in Form einer Ringerlösung oder einer gepufferten Lösung, einer wässrigen Lösung mit mindestens einem derZusätze Kohlenhydrate, ätherische Öle, Menthol- und Pflanzenextrakte, einerwässrigen Lösung, enthaltend Vitamine, Spurenelemente, Mangan oder Zink oder einer wässrigen Lösung mit mindestens einem der Zusätze aus der Gruppe umfassend Zimtöl, Teebaumöl, Salbeiöl, Thymianöl, Zitronenmelissenöl.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 und 2 zeigen eine erfindungsgemäße Inhalationseinrichtung in einer Gesamtdarstellung und einer geschnittenen Darstellung.
Fig. 3 zeigt die Komponenten der Inhalationseinrichtung in einer Explosionsdarstellung.
Fig. 4 und 5 zeigen Details des Austragkopfes.
Fig. 6A bis 6C verdeutlichen die Funktionsweise der Inhalationseinrichtung.
Fig. 7 zeigt den Sprühstrahl der Inhalationseinrichtung im Verhältnis zu der für den Austrag vorgesehenen Durchbrechung im Außengehäuse des Austragkopfes.
Fig. 8A bis 8C zeigen alternative Möglichkeiten zur Ausgestaltung des Atemstücks der Inhalationseinrichtung.
Fig. 9 zeigt eine alternative Gestaltung des Austragkopfes.

### DETAILLIERTE BESCHREIBUNG DER AUSFÜHRUNGSBEISPIELE

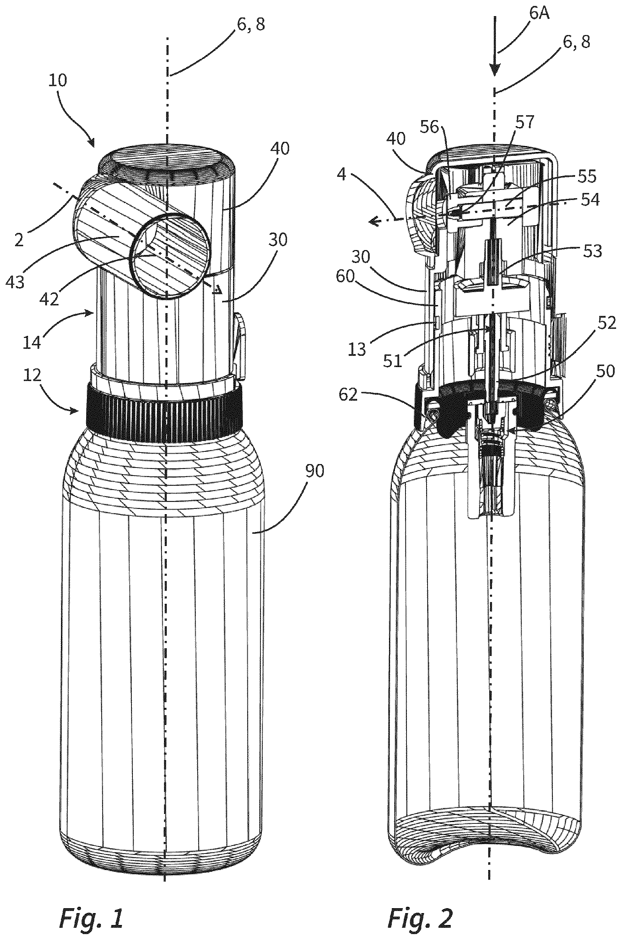

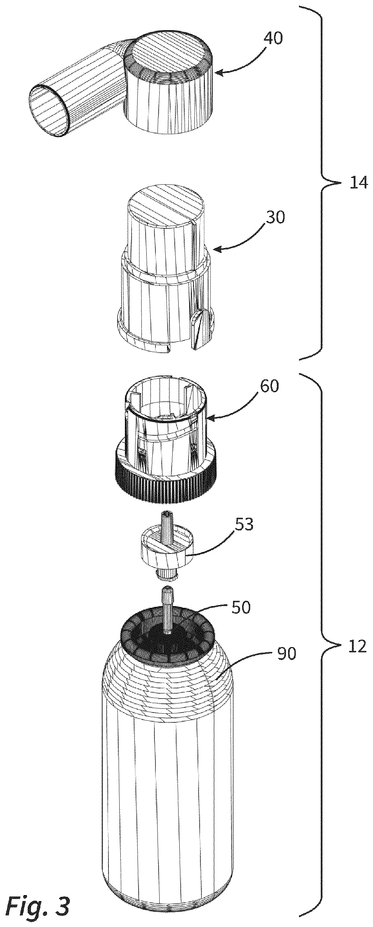

Die Fig. 1 und 2 zeigen eine erfindungsgemäße Inhalationseinrichtung 10 zum einen in einer Gesamtdarstellung und zum anderen in einer geschnittenen Darstellung. Bezug nehmend auch auf Fig. 3 weist die Inhalationseinrichtung 10 eine Basis 12 und einen gegenüber der Basis 12 um eine Hauptdrehachse 6 verdrehbaren Austragkopf 14 auf.

Die Basis 12 verfügt dabei als Hauptbestandteile über einen Flüssigkeitsspeicher 90 mit einem auslassseitigen Schaltventil 50, welches durch Verlagerung eines Stößels 52 in Richtung des Pfeils 6A geöffnet werden kann. Der Flüssigkeitsspeicher 90 ist in Art einer zu einer Mittelachse 8 rotationssymmetrischen Druckflasche ausgebildet. Die Basis 12 umfasst weiterhin ein Basisbauteil 60, welches mittels Rastmitteln 62 auf dem als Druckflasche ausgebildeten Flüssigkeitsspeicher 90 aufgeschnappt ist.

Der Austragkopf 14 verfügt als von außen sichtbare Hauptbestandteile über ein Innengehäuse 30 und ein Außengehäuse 40, wobei das Innengehäuse 30 nur teilweise vom Außengehäuse 40 überdeckt ist. Innerhalb des Innengehäuses 30 sind mehrere zur Flüssigkeitsleitung vorgesehene Bauteile 54, 55 sowie eine Düsenanordnung 56 vorgesehen, welche über eine Düsenplatte 57 aus Silizium mit einer Vielzahl im Einzelnen nicht dargestellter Düsenöffnungen verfügt.

Wie sich insbesondere anhand von Fig. 2 ersehen lässt, schließt die Abgaberichtung 4 der Düsenanordnung56 mit der Hauptdrehachse 6 der Inhalationseinrichtung 10 und der Mittelachse 8 des Flüssigkeitsspeichers einen rechten Winkel ein.

Zum Zwecke der Betätigung des Schaltventils 50 ist eine Kulissenführung 13 vorgesehen, deren helixabschnittsförmige Kulisse aus Fig. 3 ersichtlich ist. In diese Kulisse greift eine Nocke an der Innenseite des Innengehäuses 30 derart ein, dass eine Drehbewegung des Austragkopfes 14 gegenüber der Basis 12 mittelbar auch eine axiale Verlagerung des Austragkopfes 14 gegenüber der Basis 12 bewirkt, so dass mittels eines Zwischenbauteils 53 durch diese Drehbewegung der Stößel 52 niedergedrückt werden und das Schaltventil 50 geöffnet werden kann.

Wie sich anhand der Fig. 1 ersehen lässt, verfügt das Außengehäuse 40 über einen Kanalstutzen 43, der in einer Haupterstreckungsachse 2 ausgerichtet ist und einen Austragkanal 42 bildet, welcher exzentrisch zur Hauptdrehachse 6 ausgerichtet ist. Dieser Austragkanal 42 dient der Inhalation, indem der Benutzer an seinem distalen Ende einsaugt.

Bestimmungsgemäß erfolgt dies, nachdem er zuvor mit der beschriebenen Drehbewegung den Austragvorgang initiiert hat, so dass Flüssigkeit aus dem Flüssigkeitsspeicher 90 durch den Flüssigkeitskanal 51 bis zur Düsenanordnung 56 strömt und dort vernebelt wird. Das Öffnen und Schließen des Schaltventils erfolgt dabei vorzugsweise derart, dass der Benutzer den Kanalstutzen 43 oder ein hieran angebrachtes Atemstück mit seinem Mund oder seiner Gesichtspartie fixiert und dann dem Flüssigkeitsspeicher 90 um die Hauptdrehachse 6 dreht.

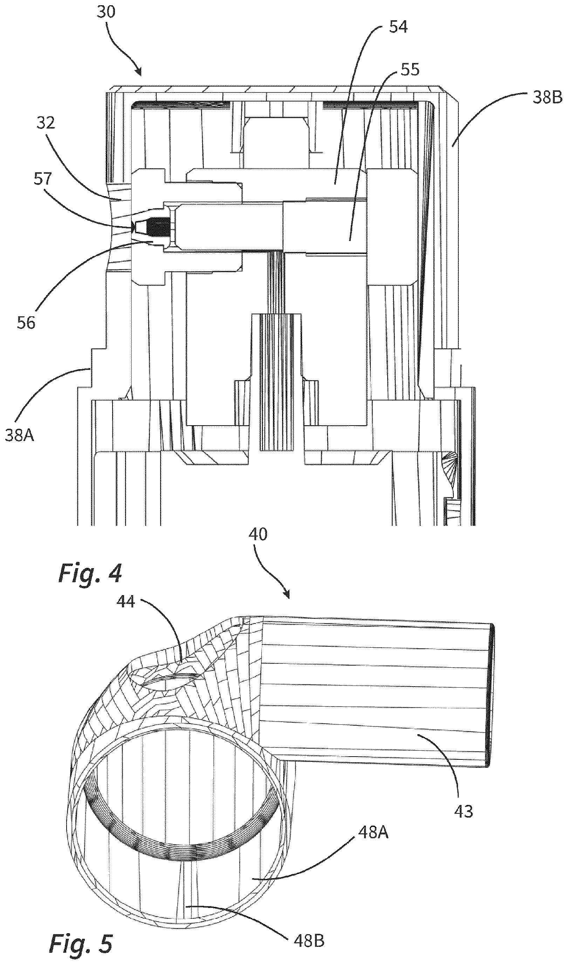

Damit das Außengehäuse 40 und das Innengehäuse 30 sich gemeinsam drehen, sind diese formschlüssig miteinander verbunden. Bezug nehmend auf die Fig. 4 und 5 ist zu erkennen, dass am Innengehäuse 30 ein Formschlusssteg 38B vorgesehen ist, der mit einer korrespondierenden Nut 48B am Außengehäuse 40 zusammenwirkt.

Es ist wichtig, dass diese konkrete Formgebung nur exemplarisch ist. Bei einer auch unter ästhetischen Gesichtspunkten gestalteten Formgebung des Außengehäuses 40 und des Innengehäuses 30 sind diese vorzugsweise als Ganzes so geformt, dass sich der gewünschte drehfeste Formschluss einstellt.

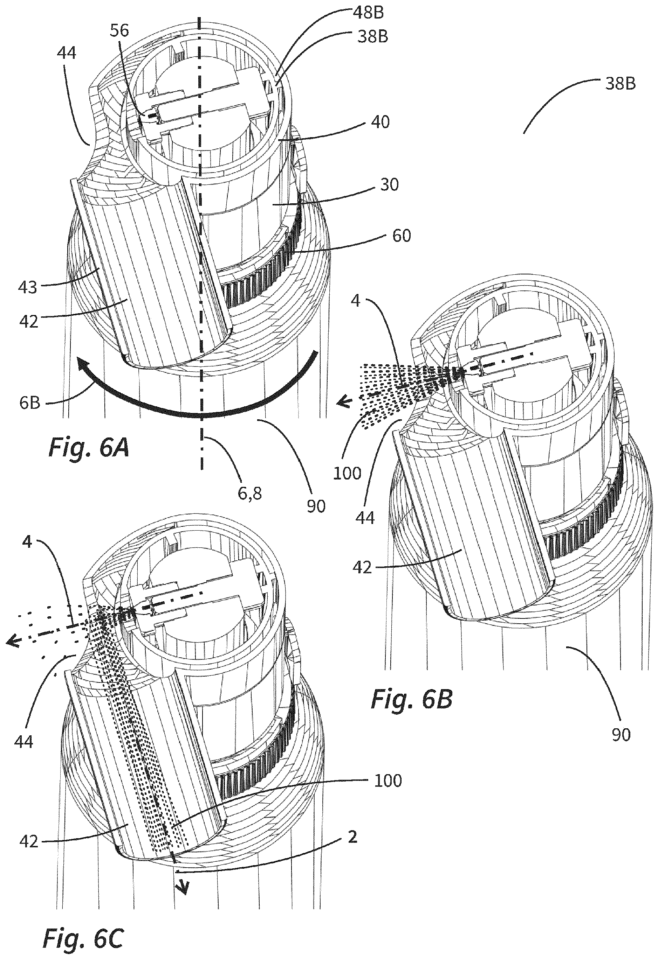

Anhand der Fig. 6A bis 6C werden die Funktionsweise und die Besonderheit des Austragkopfes 14 erläutert. Der Austragkopf 14 ist zu diesem Zweck entlang einer horizontalen Ebene auf Höhe der Düsenanordnung 56 geschnitten.

Fig. 6A zeigt den Ausgangszustand. Der Benutzer nimmt den Kanalstutzen 43 in den Mund oder fixiert ihn anderweitig mittels eines daran angebrachten Atemstücks. Anschließend wird der Flüssigkeitsspeicher 90 in die durch den Pfeil 6B verdeutlichten Weise gedreht, so dass der Austragkopf 14 und die Basis 12 sich aufgrund der Kulissenführung 13 axial aneinander annähern und der Austragkopf 14 mittelbar den Stößel 52 niederdrückt und damit das Schaltventil 50 öffnet. Die Flüssigkeit strömt nun den Flüssigkeitskanat 51 entlang bis zur Düsenanordnung 56 und wird dort in der durch Fig. 6B verdeutlichten Weise in einen Sprühnebel kleiner Tröpfchen verwandelt.

Wie Fig. 6B zeigt, kann dieser unmittelbar aus dem Austragkopf 14 durch eine mit der Düsenanordnung 56 fluchtende Durchbrechung 44 austreten. Es kommt also nicht zu einem relevanten Benetzen der Innenseiten des Austragkopfes.

Wenn der Benutzer nun durch Saugen am distalen Ende des Kanalstutzens 43 mit dem Inhalationsvorgang beginnt, stellt sich die in Fig. 6C verdeutlichte Situation ein. Die Tröpfchen des Tröpfchennebels 100 werden zum überwiegenden Teil umgelenkt und strömen dann in Richtung der Haupterstreckungsachse 2 des Austragkanals 42. Durch die Umlenkung kommt es zu einem Zerreißen der Tröpfchen, so dass der mittlere Tröpfchendurchmesser sich signifikant verringert. Wie anhand der Fig. 6C ebenfalls verdeutlicht, werden zu große Tröpfchen, die sich für die Inhalation nicht eignen würden, in geringerem Maße abgelenkt, so dass sie nicht in den Kanalstutzen 43 gelangen. Während des Inhalationsvorganges strömt die für die Verfeinerung des Tröpfchennebels 100 benötigte Luft durch die Durchbrechung44 ein.

Sobald der Benutzer seinen Einatemvorgang beendet, stellt sich wieder die Situation der Fig. 6B ein. Sofern der Benutzer durch die Inhalationseinrichtung 10 hindurch ausatmet, wird die ausgeatmete Lust gemeinsam mit dem Sprühstrahl durch die Durchbrechung 44 aus dem Außengehäuse 40 herausgeleitet.

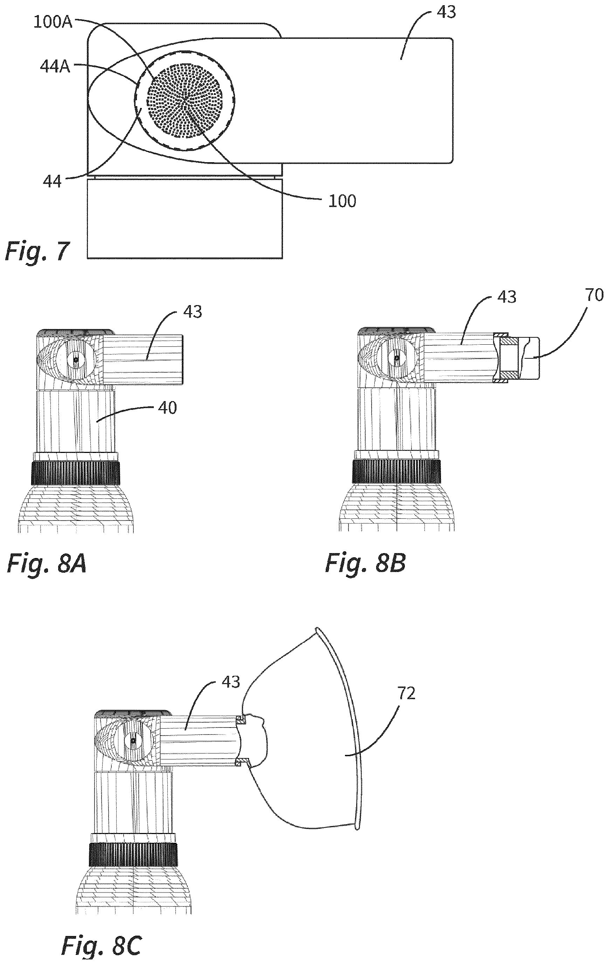

Fig. 7 verdeutlicht, dass Bezug nehmend auf den nicht abgelenkten Sprühstrahl dessen Querschnittsfläche 100A im Bereich der Durchbrechung 44 den durch die Durchbrechung 44 gebildeten Freischnitt 44A zu einem erheblichen Teil, vorliegend mehr als 50%, ausfüllt. Wenngleich dies nicht zwingend erforderlich ist, wird es doch als vorteilhaft angesehen, wenn ein derartig hoher Grad der Ausfüllung erzielt wird, da hierdurch gewährleistet ist, dass die einströmende Luft zum überwiegenden Teil an der Umlenkung des Tröpfchennebels 100 teilnimmt. Außerdem führt eine vergleichsweise kleine Durchbrechung 44 zu einer höheren Strömungsgeschwindigkeit der einströmenden Luft und somit zu einer besseren Verneblung mit kleineren Tröpfchen.

Die Fig. 8A bis 8C verdeutlichen mögliche Gestaltungen der Inhalationseinrichtung 10 mit einem Atemstück.

Fig. 8A entspricht der Gestaltung der vorangegangenen Figuren. Hier ist vorgesehen, dass der Kanalstutzen 43 unmittelbar vom Benutzer als Mundstück verwendet wird, also bestimmungsgemäß beim Inhalieren mit den Lippen umschlossen wird. Fig. 8B zeigt, dass stattdessen auch ein Einsatz in Form eines separaten Mundstücks 70 vorgesehen sein kann, beispielsweise um hierdurch die hygienische Verwendung des Inhalators durch mehrere Personen zu ermöglichen. Alternativ zu dem Mundstück 70 kann in der Fig. 8C entnehmbaren Weise auch eine separate Atemmaske 72 Verwendung finden.

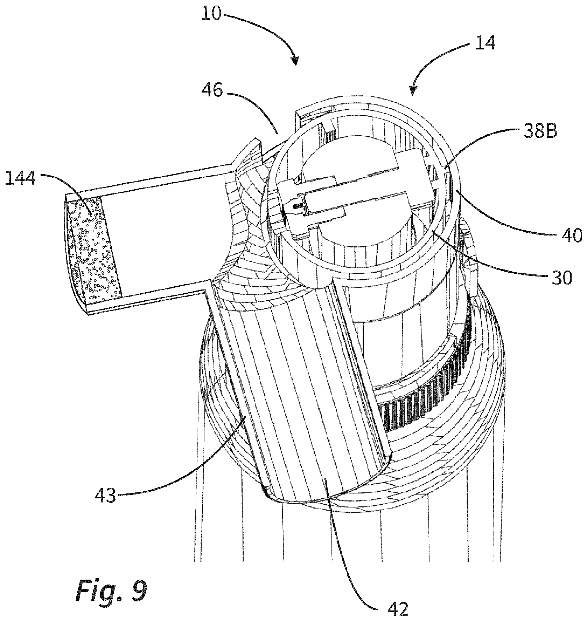

Fig. 9 zeigt eine alternative Gestaltung der Inhalationseinrichtung 10. Bei dieser ist das Außengehäuse 40 etwas anders gestaltet. Statt einer Durchbrechung44, die unmittelbar mit einer umgebenden Atmosphäre eine Verbindung schafft, findet hier ein schwammartiger oder poröser Flüssigkeitspuffer 144 Verwendung. Wird, entsprechend der Fig. 6B, nach Beginn des Austragvorgangs die Inhalation unterbrochen, so ist der Sprühstrahl auf diesen Flüssigkeitspuffer 144 gerichtet und kann hier aufgenommen werden. Nach Beendigung der Inhalation kann die Flüssigkeit dann von hier aus verdunsten. Da der Flüssigkeitspuffer bei der dargestellten Gestaltung nicht gut als Einströmkanal verwendbar ist, ist bei der Gestaltung der Fig. 9 ein separater Einströmkanal 46 vorgesehen.

## Patentansprüche

1. Inhalationseinrichtung (10) zum Zwecke des Inhalierens eines Tröpfchennebels (100) durch einen Benutzer mit den folgenden Merkmalen:
a. die Inhalationseinrichtung (10) umfasst eine Basis (12) umfassend einen Flüssigkeitsspeicher (90) zur Lagerung von Flüssigkeit vor ihrem Austrag, und
b. die Inhalationseinrichtung (10) umfasst einen Austragkopf (14) für die Abgabe der Flüssigkeit in Form eines Tröpfchennebels, und
c. der Austragkopf (14) weist eine Düsenanordnung (56) zur Erzeugung des Tröpfchennebels (100) auf, und
d. die Inhalationseinrichtung (10) umfasst ein Schaltventil (50), mittels dessen ein Zufuhrkanal vom Flüssigkeitsspeicher (90) zur Düsenanordnung (56) geöffnet und geschlossen werden kann, und
e. der Austragkopf (14) ist um eine Hauptdrehachse (6) gegenüber der Basis (12) verdrehbar, wobei hierdurch das Schaltventil (50) geöffnet und geschlossen werden kann, und
f. der Austragkopf (14) umfasst ein Innengehäuse (30), welches mit der Basis (12) verbunden ist und an welchem die Düsenanordnung (56) zur Abgabe des Tröpfchennebels (100) in einer zur Hauptdrehachse (6) angewinkelten Abgaberichtung (4) vorgesehen ist, und
g. der Austragkopf (14) umfasst ein Außengehäuse (40), welches in einer definierten Drehstellung auf das Innengehäuse (30) aufsetzbar ist, und
h. das Innengehäuse (30) und das Außengehäuse (40) sind formschlüssig in Hinblick auf eine Rotation um die Hauptdrehachse (6) zusammenwirkend ausgebildet, so dass durch eine Drehbewegung des Außengehäuses (40) gegenüber der Basis (12) mittelbar auch eine Drehbewegung des Innengehäuses (30) gegenüber der Basis (12) erzielbar ist, und
i. das Außengehäuse (40) verfügt über einen Austragkanal (42), der zur Nutzung als Atemstück oder zu Anbringung eines Atemstücks ausgebildet ist und der durch Aufsetzen des Außengehäuses (40) derart relativ zur Düsenanordnung (56) angeordnet wird, dass durch den Austragkanal (42) hindurch der von der Düsenanordnung abgegebene Tröpfchennebel (100) inhaliert werden kann, und
j. das Außengehäuse (40) weist eine Mantelfläche auf, und
k. das Außengehäuse (40) weist einen außenseitig an der Mantelfläche vorgesehenen Kanalstutzen (43) auf, der den Austragkanal (42) bildet und dessen Haupterstreckungsachse (2) gegenüber der Abgaberichtung (4) der Düsenanordnung (56) angewinkelt ist.

2. Inhalationseinrichtung (10) nach Anspruch 1 mit dem folgenden zusätzlichen Merkmal:
a. das Innengehäuse (30) und das Außengehäuse (40) weisen miteinander zusammenwirkende Klemmbereiche (38A, 48A) innenseitig am Außengehäuse (40) und außenseitig am Innengehäuse (30) auf, im Bereich derer sie unter Bildung einer Presspassung kraftschlüssig haltend ineinander geschoben werden können.

3. Inhalationseinrichtung (10) nach einem der vorstehenden Ansprüche mit dem folgenden zusätzlichen Merkmal:
a. es ist ein Flüssigkeitsaufnahmeraum (58) zur Aufnahme von Flüssigkeit vorgesehen, die sich nach Abgabe durch die Düsenanordnung (56) innenseitig am Außengehäuse (40) niederschlägt.

4. Inhalationseinrichtung (10) nach Anspruch 3 mit dem folgenden zusätzlichen Merkmal:
a. bei bestimmungsgemäßer Ausrichtung der Inhalationseinrichtung mit nach oben weisendem Austragkopf (14) und nach unten weisender Flüssigkeitsspeicher (90) ist der Flüssigkeitsaufnahmeraum (58) als vertiefter Aufnahmebereich vorgesehen, so dass in der genannten Ausrichtung keine hier gesammelte Flüssigkeit aus dem Austragkopf (14) auslaufen kann.

5. Inhalationseinrichtung (10) nach Anspruch 3 oder4 mit dem folgenden zusätzlichen Merkmal:
a. der Flüssigkeitsaufnahmeraum (58) wird gemeinsam durch einen Innenwandung des Außengehäuses (40) und eine Außenwandung des Innengehäuses (30) gebildet und/oder der Flüssigkeitsaufnahmeraum (58) ist als Ringraum ausgebildet.

6. Inhalationseinrichtung (10) nach einem der vorstehenden Ansprüche mit dem folgenden zusätzlichen Merkmal:
a. der Kanalstutzen (43), der den Austragkanal (42) bildet, endet in einem Mundstück oder in einer Atemmaske.

7. Inhalationseinrichtung (10) nach einem der Ansprüche 1 bis 5 mit dem folgenden zusätzlichen Merkmal:
a. die Inhalationseinrichtung (10) umfasst ein von dem Kanalstutzen (43), der den Austragkanal (42) bildet, getrenntes Mundstück (70) oder eine Atemmaske (72) zur Ankoppelung an den Kanalstutzen (43).

8. Inhalationseinrichtung (10) nach einem der vorstehenden Ansprüche mit dem folgenden zusätzlichen Merkmal:
a. der Flüssigkeitsspeicher (90) ist als Druckspeicher ausbildet, in dem die Flüssigkeit unter Überdruck vorgehalten wird.

9. Inhalationseinrichtung (10) nach einem der vorstehenden Ansprüche mit dem folgenden zusätzlichen Merkmal:
a. der Flüssigkeitsspeicher (90) ist zur Aufnahme von maximal 500 ml Flüssigkeit ausgelegt, vorzugsweise von maximal 250 ml.

10. Inhalationseinrichtung (10) nach einem der vorstehenden Ansprüche mit folgendem zusätzlichen Merkmal:
a. die Düsenanordnung (56) weist eine Düsenplatte (57) mit einer Mehrzahl von Düsenöffnungen auf.

11. Inhalationseinrichtung (10) nach einem der vorstehenden Ansprüche mit folgendem zusätzlichen Merkmal:
a. der Austragkopf (14) ist zum Zweck des Öffnens und Schließens des Schaltventils (50) gegenüber der Basis (12) in einer überlagerten rotativen und axialen Bewegung verlagerbar.

12. Inhalationseinrichtung (10) nach Anspruch 11 mit folgendem zusätzlichen Merkmal:
a. zwischen der Basis (12) und dem Austragkopf (14) ist eine Kulissenführung (13) mit einer helixabschnittsförmigen Kulisse vorgesehen.

13. Inhalationseinrichtung (10) nach einem der vorstehenden Ansprüche mit dem folgenden zusätzlichen Merkmal:
a. der Flüssigkeitsspeicher ist befüllt mit:
• einer salzhaltigen wässrigen Lösung oder
• einer wässrigen Lösung in Form einer Ringerlösung oder einer gepufferten Lösung oder
• einer wässrigen Lösung mit mindestens einem der Zusätze Kohlenhydrate, ätherische Öle, Menthol- und Pflanzenextrakte oder
• einer wässrigen Lösung, enthaltend Vitamine, Spurenelemente, Mangan oder Zink, oder
• einer wässrigen Lösung mit mindestens einem der Zusätze aus der Gruppe umfassend Zimtöl, Teebaumöl, Salbeiöl, Thymianöl, Zitronenmelissenöl.

## Claims

1. Inhalation device (10) for the purpose of inhalation of a droplet mist (100) by a user, having the following features:
a. the inhalation device (10) comprises a base (12) comprising a liquid reservoir (90) for storing liquid before the latter is discharged, and
b. the inhalation device (10) comprises a discharge head (14) for dispensing the liquid in the form of a droplet mist, and
c. the discharge head (14) has a nozzle arrangement (56) for generating the droplet mist (100), and
d. the inhalation device (10) comprises a switching valve (50), by means of which a delivery channel from the liquid reservoir (90) to the nozzle arrangement (56) can be opened and closed, and
e. the discharge head (14) is rotatable relative to the base (12) about a main rotation axis (6), by which means the switching valve (50) can be opened and closed, and
f. the discharge head (14) comprises an inner housing (30), which is connected to the base (12) and on which the nozzle arrangement (56) is provided for dispensing the droplet mist (100) in a dispensing direction (4) at an angle to the main rotation axis (6), and
g. the discharge head (14) comprises an outer housing (40) which can be placed onto the inner housing (30) in a defined rotation position, and
h. the inner housing (30) and the outer housing (40) interact with form-fit engagement in respect of a rotation about the main rotation axis (6), such that a rotational movement of the outer housing (40) relative to the base (12) can also directly effect a rotational movement of the inner housing (30) relative to the base (12), and
i. the outer housing (40) has a discharge channel (42), which is designed for use as a respiration piece or for attachment of a respiration piece and which, when the outer housing (40) is fitted, is arranged relative to the nozzle arrangement (56) in such a way that the droplet mist (100) dispensed from the nozzle arrangement can be inhaled through the discharge channel (42), and
j. the outer housing (40) has a jacket surface, and
k. the outer housing (40) has a channel connector (43), which is provided externally on the jacket surface (45) and forms the discharge channel (42), and of which the main axis of extent (2) is at an angle with respect to the dispensing direction (4) of the nozzle arrangement (56).

2. Inhalation device (10) according to Claim 1, having the following additional feature:
a. the inner housing (30) and the outer housing (40) have mutually interacting clamping regions (38A, 48A) on the inside of the outer housing (40) and on the outside of the inner housing (30), in the region of which they can be pushed into each other and held with force-fit engagement in order to produce an interference fit.

3. Inhalation device (10) according to one of the preceding claims, having the following additional feature:
a. a liquid-receiving space (58) is provided for receiving liquid that settles internally on the outer housing (40) after being dispensed through the nozzle arrangement (56).

4. Inhalation device (10) according to Claim 3, having the following additional feature:
a. when the inhalation device is oriented in the intended manner with the discharge head (14) facing upwards and the liquid reservoir (90) facing downwards, the liquid-receiving space (58) is provided as a recessed receiving region such that, in the stated orientation, no liquid collected here can run out of the discharge head (14) .

5. Inhalation device (10) according to Claim 3 or 4, having the following additional feature:
a. the liquid-receiving space (58) is formed jointly by an inner wall of the outer housing (40) and an outer wall of the inner housing (30), and/or the liquid-receiving space (58) is configured as an annular space.

6. Inhalation device (10) according to one of the preceding claims, having the following additional feature:
a. the channel connector (43), which forms the discharge channel (42), terminates in a mouthpiece or in a breathing mask.

7. Inhalation device (10) according to one of Claims 1 to 5, having the following additional feature:
a. the inhalation device (10) comprises a mouthpiece (70) separate from the channel connector (43) forming the discharge channel (42), or a breathing mask (72) for coupling to the channel connector (43).

8. Inhalation device (10) according to one of the preceding claims, having the following additional feature:
a. the liquid reservoir (90) is configured as a pressure accumulator in which the liquid is kept at an overpressure.

9. Inhalation device (10) according to one of the preceding claims, having the following additional feature:
a. the liquid reservoir (90) is configured to receive at most 500 ml of liquid, preferably at most 250 ml.

10. Inhalation device (10) according to one of the preceding claims, having the following additional feature:
a. the nozzle arrangement (56) has a nozzle plate (57) with a plurality of nozzle openings.

11. Inhalation device (10) according to one of the preceding claims, having the following additional feature:
a. for the purpose of opening and closing the switching valve (50), the discharge head (14) is movable relative to the base (12) in a superposed rotational and axial movement.

12. Inhalation device (10) according to Claim 11, having the following additional feature:
a. a slotted guide (13), with a slot having the shape of part of a helix, is provided between the base (12) and the discharge head (14).

13. Inhalation device (10) according to one of the preceding claims, having the following additional feature:
a. the liquid reservoir is filled with:
• a saline aqueous solution or
• an aqueous solution in the form of a Ringer's solution or a buffered solution or
• an aqueous solution with at least one of the additives carbohydrates, essential oils, menthol and plant extracts or
• an aqueous solution containing vitamins, trace elements, manganese or zinc, or
• an aqueous solution with at least one of the additives from the group comprising cinnamon oil, tea tree oil, sage oil, thyme oil, lemon balm.

## Revendications

1. Dispositif d'inhalation (10) permettant à un utilisateur d'inhaler un brouillard de gouttelettes (100), ledit dispositif présentant les caractéristiques suivantes :
a. le dispositif d'inhalation (10) comprend une base (12) munie d'un réservoir de liquide (90) destiné à stocker du liquide avant sa sortie, et
b. le dispositif d'inhalation (10) comprend une tête de sortie (14) destinée à distribuer le liquide sous la forme d'un brouillard de gouttelettes, et
c. la tête de sortie (14) comporte un ensemble formant buse (56) destiné à générer le brouillard de gouttelettes (100), et
d. le dispositif d'inhalation (10) comprend une soupape de commutation (50) permettant d'ouvrir et de fermer un conduit d'alimentation allant du réservoir de liquide (90) à l'ensemble formant buse (56), et
e. la tête de sortie (14) peut tourner sur un axe de rotation principal (6) par rapport à la base (12), la soupape de commutation (50) pouvant être ouverte et fermée, et
f. la tête de sortie (14) comprend un boîtier intérieur (30) qui est relié à la base (12) et au niveau duquel est prévu l'ensemble formant buse (56) destiné à distribuer le brouillard de gouttelettes (100) dans une direction de distribution (4) inclinée par rapport à l'axe de rotation principal (6), et
g. la tête de sortie (14) comprend un boîtier extérieur (40) qui peut être placé sur le boîtier intérieur (30) dans une position en rotation définie, et
h. le boîtier intérieur (30) et le boîtier extérieur (40) sont conçus pour coopérer par complémentarité de formes en termes de rotation sur l'axe de rotation principal (6) de sorte qu'un mouvement de rotation du boîtier extérieur (40) par rapport à la base (12) peut également entraîner indirectement un mouvement de rotation du boîtier intérieur (30) par rapport à la base (12), et
i. le boîtier extérieur (40) comporte un conduit de sortie (42) qui est conçu pour être utilisé comme pièce respiratoire ou pour monter une pièce respiratoire et qui est disposé par placement du boîtier extérieur (40) par rapport à l'ensemble formant buse (56) de manière à ce que le brouillard de gouttelettes (100) délivré par l'ensemble formant buse puisse être inhalé à travers le conduit de sortie (42), et
j. le boîtier extérieur (40) comporte une surface d'enveloppe, et
k. le boîtier extérieur (40) comporte un raccord de conduit (43) qui est prévu du côté extérieur de la surface d'enveloppe et qui forme le conduit de sortie (42) et dont l'axe principal d'extension (2) est coudé par rapport à la direction de distribution (4) de l'ensemble formant buse (56).

2. Dispositif d'inhalation (10) selon la revendication 1, ledit dispositif présentant la caractéristique supplémentaire suivante :
a. le boîtier intérieur (30) et le boîtier extérieur (40) comportent, du côté intérieur du boîtier extérieur (40) et du côté extérieur du boîtier intérieur (30), des zones de serrage (38A, 48A) qui coopèrent les unes avec les autres et au niveau desquelles ils peuvent être poussés l'un dans l'autre et maintenus en force afin de former un ajustement serré.

3. Dispositif d'inhalation (10) selon l'une des revendications précédentes, ledit dispositif présentant la caractéristique supplémentaire suivante :
a. un espace de réception de liquide (58) est prévu pour recevoir un liquide qui, après avoir été distribué par l'ensemble formant buse (56), est déposé du côté intérieur du boîtier extérieur (40).

4. Dispositif d'inhalation (10) selon la revendication 3, ledit dispositif présentant la caractéristique supplémentaire suivante :
a. lorsque le dispositif d'inhalation est correctement aligné avec la tête de sortie (14) dirigée vers le haut et le réservoir de liquide (90) dirigé vers le bas, l'espace de réception de liquide (58) est prévu comme une zone de réception évidée de sorte que, dans l'orientation susmentionnée, aucun liquide collecté ici ne puisse s'écouler de la tête de sortie (14).

5. Dispositif d'inhalation (10) selon la revendication 3 ou 4, ledit dispositif présentant la caractéristique supplémentaire suivante :
a. l'espace de réception de liquide (58) est formé conjointement par une paroi intérieure du boîtier extérieur (40) et une paroi extérieure du boîtier intérieur (30) et/ou l'espace de réception de liquide (58) est conçu comme un espace annulaire.

6. Dispositif d'inhalation (10) selon l'une des revendications précédentes, ledit dispositif présentant la caractéristique supplémentaire suivante :
a. le raccord de conduit (43), qui forme le conduit de sortie (42), se termine par un embout buccal ou par un masque respiratoire.

7. Dispositif d'inhalation (10) selon l'une des revendications 1 à 5, ledit dispositif présentant la caractéristique supplémentaire suivante :
a. le dispositif d'inhalation (10) comprend un embout buccal (70), séparé du raccord de conduit (43) qui forme le conduit de sortie (42), ou un masque respiratoire (72) destiné à s'accoupler au raccord de conduit (43).

8. Dispositif d'inhalation (10) selon l'une des revendications précédentes, ledit dispositif présentant la caractéristique supplémentaire suivante :
a. le réservoir de liquide (90) est conçu comme un réservoir sous pression dans lequel le liquide est maintenu en surpression.

9. Dispositif d'inhalation (10) selon l'une des revendications précédentes, ledit dispositif présentant la caractéristique supplémentaire suivante :
a. le réservoir de liquide (90) est conçu pour contenir au maximum 500 ml de liquide, de préférence au maximum 250 ml.

10. Dispositif d'inhalation (10) selon l'une des revendications précédentes, ledit dispositif présentant la caractéristique supplémentaire suivante :
a. l'ensemble formant buse (56) comporte une plaque formant buse (57) munie d'une pluralité d'ouvertures formant buses.

11. Dispositif d'inhalation (10) selon l'une des revendications précédentes, ledit dispositif présentant la caractéristique supplémentaire suivante :
a. la tête de sortie (14) est déplaçable par rapport à la base (12) suivant un mouvement rotatif et un mouvement axial superposés afin d'ouvrir et de fermer la soupape de commutation (50).

12. Dispositif d'inhalation (10) selon la revendication 11 ledit dispositif présentant la caractéristique supplémentaire suivante :
a. un guide à coulisse (13) pourvu d'une coulisse en forme de segment hélicoïdal est prévu entre la base (12) et la tête de sortie (14).

13. Dispositif d'inhalation (10) selon l'une des revendications précédentes, ledit dispositif présentant la caractéristique supplémentaire suivante :
a. le réservoir de liquide est rempli :
• d'une solution aqueuse saline ou
• d'une solution aqueuse sous forme de solution de Ringer ou de solution tamponnée ou
• d'une solution aqueuse comportant l'un au moins des additifs glucides, huiles essentielles, menthol et extraits de plantes ou
• d'une solution aqueuse contenant des vitamines, des oligo-éléments, du manganèse ou du zinc, ou
• d'une solution aqueuse comportant l'un au moins des additifs du groupe comprenant l'huile de cannelle, l'huile d'arbre à thé, l'huile de sauge, l'huile de thym, l'huile de mélisse et de citronnelle.
